# EUROPÄISCHE PATENTSCHRIFT

(11) **EP 0 510 491 B1**
(45) Veröffentlichungstag und Bekanntmachung des Hinweises auf die Patenterteilung: **03.08.1994**
(21) Anmeldenummer: 92106419.2
(22) Anmeldetag: 14.04.1992
(51) Int. Cl.: C07D 209/48, C07C 63/70, C07C 63/68

(54) **N'-substituierte N-Amino-3,4,5,6-tetrafluorphthalimide und Verfahren zu ihrer Herstellung**
N'-substituted N-amino-3,4,5,6-tetrafluorophthalimides and processes for their preparation
N'-substituée N-amino-3,4,5,6-tetrafluorophthalimides et procédés pour leur préparation

(30) Priorität: 25.04.1991 DE 4113461
(43) Veröffentlichungstag der Anmeldung: 28.10.1992
(73) Patentinhaber: HOECHST AKTIENGESELLSCHAFT, 65926 Frankfurt am Main (DE)
(72) Erfinder: Papenfuhs, Theodor, Dr., W-6000 Frankfurt am Main 50 (DE); Pfirmann, Ralf, Dr., W-6103 Griesheim (DE)

(56) Entgegenhaltungen:
- EP-A- 0 194 671
- EP-A- 0 218 111
- EP-A- 0 259 663
- DE-A- 3 810 093
- CHEMICAL ABSTRACTS, vol. 113, no. 17, 22. Oktober 1990, Columbus, Ohio, US;abstract no. 152040C, 'Preparation of fluorobenzoic acids and their intermidiate fluorophthalimides.'

## Beschreibung

Die vorliegende Erfindung betrifft neue N′-substituierte N-Amino-3,4,5,6-tetrafluorphthalimide und Verfahren zu ihrer Herstellung. Die neuen Verbindungen können durch saure Hydrolyse oder Alkoholyse in die 2,3,4,5-Tetrafluorbenzoesäure, die ein wichtiges Vorprodukt zur Herstellung antibakterieller Mittel darstellt, umgewandelt werden [DE-A-33 18 145].

Tetrafluorbenzoesäure konnte bisher aus Tetrachlorphthaloylchlorid (G.G. Yakobson, V.N. Odinkov, N.N. Vorozhtsov, Zh. Obshsh. Khim. 36 (1966), 139; Imperial Chemical Industries PLC, EP 140 482, GB 2 146 635, 24.7.84), aus Tetrafluoranthranilsäure (S. Hayashi, N. Ishikawa, Bull. Chem. Soc. Jap. 45 (1972), 2909), aus 1,2,3,4-Tetrafluorbenzol (L.J. Belf, M.W. Buxton, J.F. Tilney-Bassett, Tetrahedron 23 (1967), 4719; Z. Naturforsch. 31B (1976), 1667), aus Tetrachlorphthalsäureanhydrid (Bayer AG, DE 3 810 093 A1, 5.10.89; Warner-Lambert Co., EP 218 111, 9.9.86) oder aus Tetrachlorphthalodinitril (Imperial Chemical Industries PLC, GB 2 134 900, 22.8.84) über teilweise aufwendige und/oder technisch nicht zu verwirklichende Schritte dargestellt werden. Dieselbe Aussage kann für die Herstellung der Tetrafluorbenzoesäure aus 1,2-Dibromtetrafluorbenzol (C. Tamborski, E.J. Soloski, J. Organometallic Chem., 10 (1967), 385) und die von P. Sartori und A. Golloch (Chem. Ber. 101 (1968), 2004) beschriebene Methode, ausgehend von Tetrafluorphthalsäure, getroffen werden. Ebenfalls eingesetzt wurden N-kohlenstoffsubstituierte Tetrachlorphthalimide zur Synthese von Tetrafluorphthalsäure (SDS Biotech. K.K., EP 259 663, 18.8.87), die in 2,3,4,5-Tetrafluorbenzoesäure umgewandelt werden kann.

Aus Tetrafluorphthalsäure bzw. deren Anhydrid kann 2,3,4,5-Tetrafluorbenzoesäure nach verschiedenen Verfahren erhalten werden (EP 194 671; EP 218 111; JP 01/025 737; JP 63/295 529). Diese Verfahren laufen zum Teil unter Verwendung technisch nicht verfügbarer oder ökologisch bedenklicher Reagenzien ab. Das Hauptproblem stellt meist die Notwendigkeit der Isolierung von Tetrafluorphthalsäure dar, was erhebliche Schwierigkeiten bereiten kann, bevor diese weiter umgesetzt wird.

Es bestand somit ein Bedürfnis nach einer besseren Herstellungsmöglichkeit für das Vorprodukt 2,3,4,5-Tetrafluorbenzoesäure, welches dadurch befriedigt werden konnte, daß erfindungsgemäß N′-substituierte N-Amino-3,4,5,6-tetrafluorphthalimide hergestellt werden können, die ihrerseits, wie vorstehend erwähnt, in an sich bekannter Weise in die 2,3,4,5-Tetrafluorbenzoesäure überführt werden können.

Gegenstand der vorliegenden Erfindung sind neue N′-substituierte N-Amino-3,4,5,6-tetrafluorphthalimide der allgemeinen Formel (1) 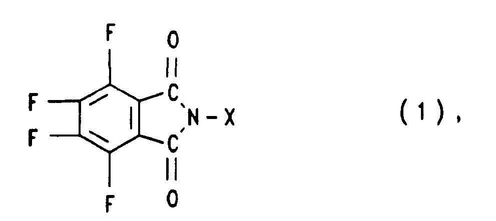
in welcher X den Rest 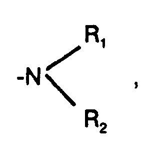
worin R₁, R₂ je ein Wasserstoffatom, eine Alkyl-(C₁-C₁₀)-Gruppe, Arylgruppe, beispielsweise die Phenylgruppe, eine Alkyl (C₁-C₆)-CO-Gruppe, beispielsweise die Acetylgruppe, eine Aryl-CO-Gruppe, beispielsweise die Benzoylgruppe, bedeuten, wobei die Aryl- bzw. Aryl-CO-Gruppen für R₁ und R₂ am aromatischen Kern beispielsweise durch Fluor- und/oder Chloratome und/oder Alkyl-(C₁-C₄)-Gruppen substituiert sein können, oder R₁ und R₂ zusammen einen Phthaloylrest, der am aromatischen Ring durch 4 Chloratome oder 4 Fluoratome substituiert sein kann, vorzugsweise den Rest 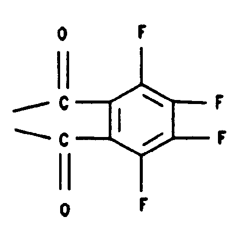
bedeuten, oder in welcher X den Rest 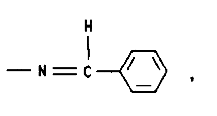
welcher am aromatischen Kern beispielsweise durch Fluor- und/oder Chloratome und/oder Alkyl-(C₁-C₄)-Gruppen substituiert sein kann, bedeutet, sowie ein Verfahren zu ihrer Herstellung, indem man 1 Mol 3,4,5,6-Tetrachlorphthalsäureanhydrid mit der mindestens äquimolaren Menge, zweckmäßigerweise mit einem molaren Überschuß bis etwa 20 Mol.-%, einer Stickstoffverbindung der allgemeinen Formel (2) 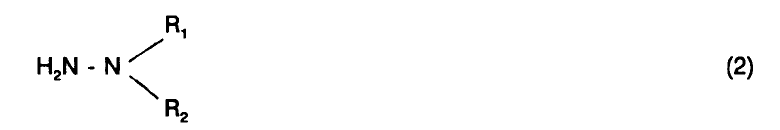
in welcher R₁ und R₂ die vorstehend genannten Bedeutungen haben, in wäßrigalkoholischem Medium, in Eisessig, in etwa 90 bis 100 %iger Schwefelsäure oder in Oleum bei Temperaturen (in Abhängigkeit vom angewandten Medium) von etwa 100 bis etwa 220°C zum entsprechenden N′-substituierten N-Amino-3,4,5,6-tetrachlorphthalimid der allgemeinen Formel (3) 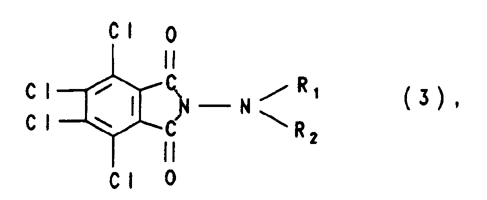
in welcher R₁ und R₂ die vorstehend genannten Bedeutungen haben, umsetzt, das so erhaltene Imid der genannten Formel (3) direkt oder nach erfolgter Umsetzung mit der mindestens äquimolaren Menge Benzaldehyd, welcher am aromatischen Kern beispielsweise durch Fluor- und/oder Chloratome und/oder Alkyl-(C₁-C₄)-Gruppen substituiert sein kann, in an sich bekannter Weise zur entsprechenden Benzalverbindung, oder nach erfolgter Acylierung mit einem Alkyl-(C₁-C₆)-CO-halogenid, vorzugsweise -chlorid, Carbonsäureanhydrid der allgemeinen Formel Alkyl(C₁-C₆)-CO-O-OC-(C₁-C₆)Alkyl, einem Aryl-CO-halogenid, vorzugsweise -chlorid, oder Phthalsäureanhydrid, das am aromatischen Ring durch 4 Chloratome oder 4 Fluoratome substituiert sein kann, in an sich bekannter Weise, in einem polar aprotischen Lösungsmittel mit Kalium-, Rubidium- oder Cäsiumfluorid oder Mischungen daraus, vorzugsweise mit Kaliumfluorid allein, bei Temperaturen von etwa 50 bis etwa 230°C, vorzugsweise etwa 90 bis etwa 140°C, in Gegenwart oder Abwesenheit eines Phasentransferkatalysators umsetzt (Halex-Reaktion).

Es versteht sich von selbst, daß im Falle einer vorausgehenden Umsetzung des Imids der allgemeinen Formel (3) mit dem gegebenenfalls kernsubstituierten Benzaldehyd zur Benzalverbindung der Formel (4) 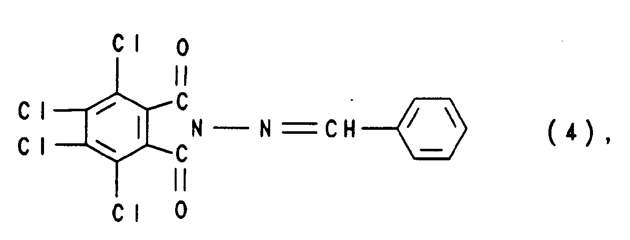
R₁ und R₂ in den Verbindungen der Formeln (2) und (3) gleichzeitig Wasserstoffatome bedeuten, und im Falle einer vorausgehenden Acylierung des Imids der allgemeinen Formel (3) mindestens einer der Reste R₁ und R₂ in den Verbindungen der Formeln (2) und (3) ein Wasserstoffatom darstellt.

Die genannten Alkalimetallfluoride werden in Mengen von 100 bis etwa 500 Mol.-%, vorzugsweise von etwa 101 bis etwa 150 Mol-%, besonders bevorzugt von etwa 102 bis etwa 120 Mol-% pro auszutauschendem Chloratom eingesetzt. Bei 4 auszutauschenden Chloratomen pro Molekül setzt man besonders bevorzugt etwa 4,08 bis etwa 4,8 Equivalente an den genannten Alkalimetallfluoriden, gegebenenfalls in Mischung, ein.

Geeignete polar aprotische Lösungsmittel für die Fluorierung (Halex-Reaktion) sind beispielsweise Dimethylformamid, Dimethylacetamid, Dimethylsulfoxid, Tetramethylensulfoxid, Dimethylsulfon, Diphenylsulfoxid, Diphenylsulfon, Sulfolan, N-Methylpyrrolidon oder 1,3-Dimethylimidazolidin-2-on.

Als Phasentransferkatalysatoren können beispielsweise quartäre Ammonium- oder Phosphoniumsalze dienen. An geeigneten Verbindungen seien im einzelnen folgende genannt: Tetraalkyl-(C₁-C₁₈)-ammoniumchloride oder -bromide, Tetraalkyl-(C₁-C₁₈)-phosphoniumchloride oder -bromide, Tetraphenylphosphoniumchlorid oder -bromid, [(Phenyl)ₘ(alkyl(C₁-C₁₈))ₙ]-phosphoniumchloride oder -bromide, wobei m = 1 bis 3, n = 3 bis 1 und m + n = 4 ist.

Die Phasentransferkatalysatoren verwendet man in Mengen von etwa 0,1 bis etwa 50 Mol-%, vorzugsweise von etwa 1 bis etwa 20 Mol-%, besonders bevorzugt von etwa 2,5 bis etwa 15 Mol-%, bezogen auf das N′-substituierte N-Amino-3,4,5,6-tetrachlorphthalimid der genannten Formel (3) bzw. (4).

Die Verfahrensdurchführung in Abwesenheit von Phasentransferkatalysatoren ist bevorzugt.

Das gegebenenfalls in erster Stufe eingesetzte Oleum enthält zweckmäßigerweise 0 bis etwa 50 %, vorzugsweise etwa 0,5 bis etwa 15 % SO₃.

Die gegebenenfalls vor der Halex-Reaktion erfolgende Umwandlung des Imids der genannten allgemeinen Formel (3) mit dem gegebenenfalls kernsubstituierten Benzaldehyd erfolgt in an sich bekannter Weise [HOUBEN WEYL Band 10/2, Seiten 89-97, und Band 11/2, Seiten 73-85 und 89-99].

Die gegebenenfalls vor der Halex-Reaktion erfolgende Acylierung des Imids der genannten allgemeinen Formel (3) kann nach an sich bekannten Methoden vorgenommen werden. So können Acylgruppen durch Reaktion mit einem oder zwei freien Wasserstoffatomen für R₁ und/oder R₂ des Imids der allgemeinen Formel (3) in einem inerten Lösungsmittel, wie beispielsweise Wasser, sauren oder alkalischen wäßrigen Lösungen, Methylenchlorid, Chloroform, Toluol, Xylole oder Chlorbenzol, unter Anwendung von etwa 0,8 bis etwa 5 Äquivalenten Base bei Temperaturen von etwa 0 bis etwa 200°C mit Acylhalogeniden - bevorzugt Acylchloriden - eingeführt werden. Ebenso gelingt die Umsetzung mit Acylanhydriden in äquimolaren Mengen bis zu großen Überschüssen mit oder ohne Lösungsmittel ohne Anwesenheit von Basen bei Temperaturen von etwa 0 bis etwa 200°C, vorzugsweise etwa 80 bis etwa 150°C. Weitere ebenfalls bekannte und in den nachstehend zitierten Literatustellen angegebene Acylierungsvarianten bieten sich an [HOUBEN WEYL, Methoden der organischen Chemie, Band 8, Seiten 655 - 661 (1952); Band 10/2, Seiten 127 - 168 (1967); Band 11/2, Seiten 3 - 38 (1958); Band E5/2, Seiten 934 - 1129, insbesondere Seiten 932 - 981 und 1116 - 1121 (1985)].

Die erfindungsgemäß erhaltenen Verbindungen der genannten allgemeinen Formel (1) können durch Hydrolyse mit wäßriger Mineralsäure in an sich bekannter Weise in das 3,4,5,6-Tetrafluorphthalsäureanhydrid bzw. durch Alkoholyse mit alkoholischer Mineralsäure in an sich bekannter Weise in die entsprechenden 3,4,5,6-Tetrafluorphthalsäurediester überführt werden, die ihrerseits nach literaturbekannten Methoden durch weitere Hydrolyse bzw. Decarboxylierung in die 2,3,4,5-Tetrafluorbenzoesäure umgewandelt werden können.

Das vorliegende Verfahren besitzt als wesentlichen Vorteil gegenüber bekannten Verfahren die günstige Verfügbarkeit der Ausgangsstoffe. Hinzu kommt, daß bei der Hydrolyse der Zwischenprodukte keine erhebliche Fluorwasserstoffentwicklung auftritt, weil keine aliphatisch gebundenen Fluoratome eingeführt wurden, wodurch das bei einigen bekannten Verfahren zu erwartende Werkstoffproblem umgangen wird. Ferner können durch die hohe Reaktivität der Tetrachlorverbindungen relativ niedrige Reaktionstemperaturen angewendet werden, wodurch Zersetzungen weitgehend unterbleiben.

Analog können auch Mono-, Di- und Trifluorphthalimide hergestellt werden.

Das erfindungsgemäße Verfahren kann sowohl bei Atmosphärendruck als auch bei Unter- oder Überdruck durchgeführt werden.

Die nachstehenden Beispiele dienen zur Erläuterung des erfindungsgemäßen Verfahrens, ohne es darauf zu beschränken.

### Beispiel 1

285,9 g (1 Mol) Tetrachlorphthalsäureanhydrid werden mit 50,1 g (1 Mol) Hydrazinhydrat in 250 ml Wasser/250 ml Ethanol 2 Stunden zum Sieden erhitzt. Nach Entfernen des Ethanols läßt man abkühlen und saugt anschließend das erhaltene N-Aminotetrachlorphthalimid ab (Ausbeute 266,9 g, 0,89 Mol, 89 %). Dieses wird mit 95,9 g (0,90 Mol) Benzaldehyd in 500 ml Eisessig bei 110°C 3 Stunden gerührt, wobei die gelbe Benzalverbindung entsteht, die nach dem Abkühlen abgesaugt und getrocknet werden kann. (Ausbeute 303,9 g, 0,783 Mol, 88 %). Nach Eintragen der Benzalverbindung in 800 ml Sulfolan bei 160°C werden 206,2 g (3,5 Mol) Kaliumfluorid zugegeben. Nach 12 Stunden bei dieser Temperatur sind alle Chloratome gegen Fluoratome ausgetauscht, wie durch GC-Kontrolle nachgewiesen werden kann. Man läßt abkühlen, saugt das Salz ab und wäscht mit Sulfolan nach. Nach Entfernen von ca. 80 % des Sulfolans bei 2 - 3 Torr wird der Rückstand (ca. 400 ml) unter Rühren in 600 ml Wasser gegeben. Man saugt den ausgefallenen Feststoff ab und wäscht zweimal mit je 100 ml Wasser nach. Nach dem Trocknen im Vakuum erhält man 161,1 g (0,501 Mol; 64 %) N-(N′-Benzyliden)aminotetrafluorphthalimid als beigefarbene, kristalline Substanz (Mischung des cis-trans-Isomeren).
Schmelzpunkt: 179 - 186°C
¹H-NMR (CDCl₃, interner Standard TMS):
- δ =: 7.45 (m, 3,2H, Ar-H)
7.86 (m, 2,8H, Ar-H)
8.65 (s, 0,63H, -N=CH-)
9.28 (s, 0,57H, -N=CH-)

¹⁹F-NMR (Aceton, interner Standard CFCl₃):
- δ =: -135.3 (2ddd, 2F); -141.7 (2ddd, 2F)

- MS: m/z (%) =: 76 (13), 90 (19), 103 (100), 146 (41); 177 (39), 207 (13), 219 (15), 281 (4), 322 (M⁺, 12)

### Beispiel 2

285,9 g (1 Mol) Tetrachlorphthalsäureanhydrid werden mit 50,1 g (1 Mol) Hydranzinhydrat in 250 ml Wasser/250 ml Ethanol 2 Stunden zum Sieden erhitzt. Nach Entfernen des Ethanols läßt man abkühlen und saugt anschließend das erhaltene N-Aminotetrachlorphthalimid ab, das in äquimolarer Menge mit Tetrachlorphthalsäureanhydrid in siedendem Eisessig umgesetzt wird. Man erhält das N-Tetrachlorphthalimidotetrachlorphthalimid als farblose, kristalline Masse in 91 %iger Ausbeute nach Abkühlen und Absaugen. Davon werden 567,8 g (1 Mol) in 1,5 l N,N-Dimethylacetamid suspendiert und die Mischung auf 100°C geheizt. Nach Zugabe eines Gemisches von 500 g (8,6 Mol) Kaliumfluorid und 50 g (0,32 Mol) Cäsiumfluorid wird 5 Stunden bei dieser Temperatur gerührt, das Salz abgesaugt und der Großteil des Lösungsmittels im Vakuum abgezogen. Man gibt auf 1 l Wasser und saugt das ausgefallene Rohprodukt ab, das zweimal mit 100 ml Wasser vom Lösungsmittel frei gewaschen wird. Es werden 302,3 g (0,693 Mol, 69 %) N-Tetrafluorphthalimidotetrafluorphthalimid erhalten, das in Form ockerfarbener Kristalle anfällt, aus n-Hexan/Xylol aber farblos kristallisiert werden kann.
Schmelzpunkt: 311 - 312°C
¹⁹F-NMR (Aceton, interner Standard CFCl₃):
- δ =: -135.0 (ddd, 4F); -142.6 (ddd, 4F)

- IR [cm⁻¹]:*v* =: (s) 1760, 1510, 1405, 1285, 1080, 950, 740, 625
(w) 1645, 1320, 1160, 1145, 1120, 915
- MS: m/z (%) =: 79 (3), 98 (9), 148 (69), 176 (70), 202 (6), 281 (1), 324 (3), 373 (3), 392 (28), 436 (M⁺, 100)

### Beispiel 3

In 500 ml Eisessig werden 285,9 g (1 Mol) Tetrachlorphthalsäureanhydrid und 60,1 g (1 Mol) N,N-Dimethylhydrazin 4 Stunden auf 60°C erhitzt. Man saugt die kalte Suspension ab, trocknet im Vakuum und erhält 309,8 g (0,945 Mol) N′,N′-Dimethylaminotetrachlorphthalimid, das in 800 g N-Methylpyrrolidon aufgenommen und mit einer Mischung aus Kaliumfluorid und Cäsiumfluorid (280,9 g, 4,54 Mol) 4 Stunden bei 160°C gehalten wird. Anschließend saugt man das Reaktionssalz ab und entfernt den größten Teil (550 g) des Lösungsmittels im Vakuum. Nach Versetzen des Rückstandes mit 900 g Wasser wird der ausgefallene Feststoff abgesaugt, welcher nach Trocknen 173,5 g (0,662 Mol, 70 %) N,N-Dimethylaminotetrafluorphthalimid in Form eines beigefarbenen Festkörpers ergibt.
Schmelzpunkt: 184 - 189°C.
¹H-NMR (CDCl₃, interner Standard TMS):
- δ =: 3.00 (s, 6H, -N(CH₃)₂)

¹⁹F-NMR (CDCl₃, interner Standard CFCl₃):
- δ =: -135.8 (ddd, 2F); -142.5 (ddd, 2F)

- MS: m/z (%) =: 43 (100), 76 (6), 98 (9), 148 (34), 176 (10), 202 (22), 221 (41), 262 (M⁺, 22)

## Patentansprüche (Patentansprüche für folgende(n) Vertragsstaat(en): BE, CH, DE, FR, GB, IT, LI, NL)

1. Verbindungen der allgemeinen Formel 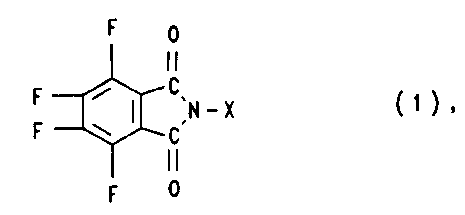 in welcher X den Rest 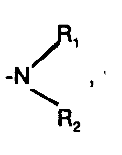 worin R₁, R₂ je ein Wasserstoffatom, eine Alkyl(C₁-C₁₀)-Gruppe, Arylgruppe, Alkyl(C₁-C₆)-CO-Gruppe oder Aryl-CO-Gruppe bedeuten, wobei die Aryl- bzw. Aryl-CO-Gruppen für R₁ und R₂ am aromatischen Kern durch Fluor- und/oder Chloratome und/oder Alkyl(C₁-C₄)-Gruppen substituiert sein können, oder R₁ und R₂ zusammen einen Phthaloylrest, der am aromatischen Ring durch 4 Chloratome oder 4 Fluoratome substituiert sein kann, vorzugsweise den Rest 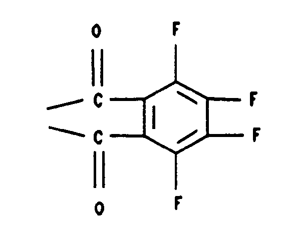 bedeuten, oder in welcher X den Rest 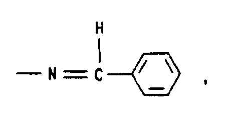 bedeutet,
welcher am aromatischen Kern durch Fluor- und/oder Chloratome und/oder Alkyl(C₁-C₄)-Gruppen substituiert sein kann.

2. Verbindung der Formel

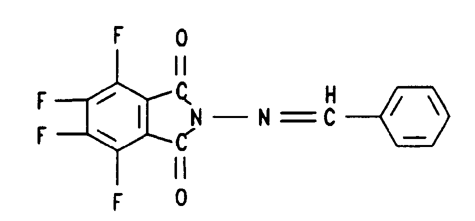

3. Verbindung der Formel

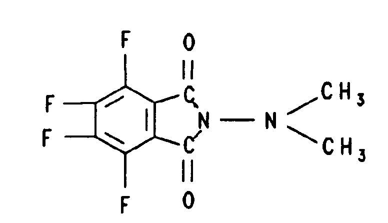

4. Verbindung der Formel

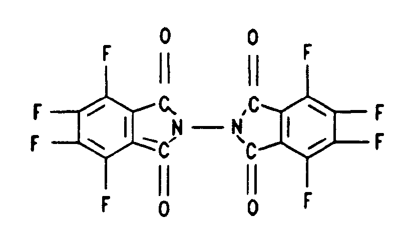

5. Verfahren zur Herstellung von Verbindungen der allgemeinen Formel (1) 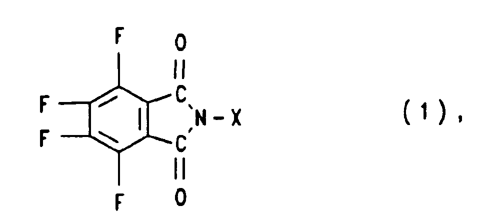 in welcher X den Rest 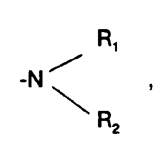 worin R₁, R₂ je ein Wasserstoffatom, eine Alkyl(C₁-C₁₀)-Gruppe, Arylgruppe, Alkyl(C₁-C₆)-CO-Gruppe oder Aryl-CO-Gruppe bedeuten, wobei die Aryl- bzw. Aryl-CO-Gruppen für R₁ und R₂ am aromatischen Kern durch Fluor- und/oder Chloratome und/oder Alkyl(C₁-C₄)-Gruppen substituiert sein können, oder R₁ und R₂ zusammen einen Phthaloylrest, der am aromatischen Ring durch 4 Chloratome oder 4 Fluoratome substituiert sein kann, vorzugsweise den Rest 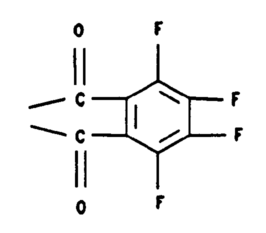 bedeuten, oder in welcher X den Rest 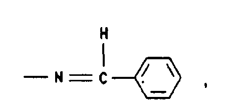 welcher am aromatischen Kern durch Fluor- und/oder Chloratome und/oder Alkyl(C₁-C₄)-Gruppen substituiert sein kann, bedeutet, dadurch gekennzeichnet, daß man 1 Mol 3,4,5,6-Tetrachlorphthalsäureanhydrid mit der mindestens äquimolaren Menge einer Stickstoffverbindung der allgemeinen Formel (2) 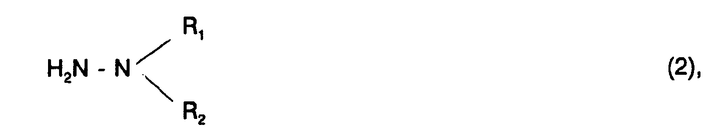 in welcher R₁ und R₂ die vorstehend genannten Bedeutungen haben, in wäßrig-alkoholischem Medium, in Eisessig, in etwa 90 bis 100 %iger Schwefelsäure oder in Oleum bei Temperaturen (in Abhängigkeit vom angewandten Medium) von etwa 100 bis etwa 220°C zum entsprechenden N′-substituierten N-Amino-3,4,5,6-tetrachlorphthalimid der allgemeinen Formel (3) 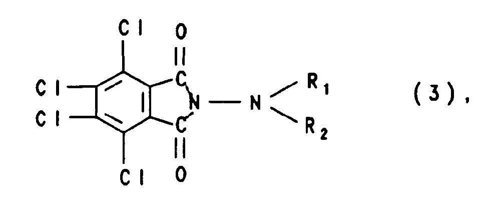 in welcher R₁ und R₂ die vorstehend genannten Bedeutungen haben, umsetzt, das so erhaltene Imid direkt oder nach erfolger Umsetzung mit der mindestens äquimolaren Menge Benzaldehyd, welcher am aromatischen Kern durch Fluor- und/oder Chloratome und/oder Alkyl(C₁-C₄)-Gruppen substituiert sein kann, zur entsprechenden Benzalverbindung, oder nach erfolgter Acylierung mit einem Alkyl(C₁-C₆)-CO-halogenid, Carbonsäureanhydrid der allgemeinen Formel Alkyl(C₁-C₆)-CO-O-OC-(C₁-C₆)Alkyl, Aryl-CO-halogenid oder Phthalsäureanhydrid, das am aromatischen Ring durch 4 Chloratome oder 4 Fluoratome substituiert sein kann, in einem polaren aprotischen Lösungsmittel mit Kalium-, Rubidium- oder Cäsiumfluorid oder Mischungen daraus bei Temperaturen von etwa 50 bis etwa 230°C in Gegenwart oder Abwesenheit eines Phasentransferkatalysators umsetzt.

6. Verfahren nach Anspruch 5, dadurch gekennzeichnet, daß man die Umsetzung mit Kalium-, Rubidium- oder Cäsiumfluorid oder Mischungen daraus bei Temperaturen von etwa 90 bis etwa 140°C vornimmt.

7. Verfahren nach mindestens einem der Ansprüche 5 und 6, dadurch gekennzeichnet, daß man die genannten Alkalimetallfluoride in Mengen von 100 bis etwa 500 Mol-% pro auszutauschendem Chloratom einsetzt.

8. Verfahren nach mindestens einem der Ansprüche 5 bis 7, dadurch gekennzeichnet, daß man die Fluorierung in Dimethylformamid, Dimethylacetamid, Dimethylsulfoxid, Tetramethylensulfoxid, Dimethylsuffon, Diphenylsulfoxid, Diphenylsulfon, Sulfolan, N-Methylpyrrolidon oder 1,3-Dimethylimidazolidin-2-on als polar aprotischem Lösungsmittel vornimmt.

9. Verfahren nach mindestens einem der Ansprüche 5 bis 8, dadurch gekennzeichnet, daß man die Fluorierung in Gegenwart eines quartären Ammonium- oder Phosphoniumsalzes als Phasentransferkatalysator vornimmt.

10. Verfahren nach mindestens einem der Ansprüche 5 bis 9, dadurch gekennzeichnet, daß man die Fluorierung in Gegenwart von Tetraalkyl(C₁-C₁₈)-ammoniumchloriden oder -bromiden, Tetraalkyl(C₁-C₁₈)-phosphoniumchloriden oder -bromiden, Tetraphenylphosphoniumchlorid oder -bromid, [(Phenyl)ₘ(alkyl(C₁-C₁₈))ₙ]-phosphonium-chloriden oder -bromiden, wobei m = 1 bis 3, n = 3 bis 1 und m + n = 4 ist, als Phasentransferkatalysatoren vornimmt.

11. Verfahren nach mindestens einem der Ansprüche 5 bis 10, dadurch gekennzeichnet, daß man die Phasentransferkatalysatoren in Mengen von etwa 0,1 bis etwa 50 Mol-%, bezogen auf das N′-substituierte N-Amino-3,4,5,6-tetrachlorphthalimid, einsetzt.

12. Verfahren nach mindestens einem der Ansprüche 5 bis 11, dadurch gekennzeichnet, daß man bei Atmosphärendruck, Über- oder Unterdruck arbeitet.

13. Verfahren zur Herstellung von 2,3,4,5-Tetrafluorbenzoesäure, dadurch gekennzeichnet, daß man die nach Ansprüchen 5 bis 12 hergestellten Verbindungen der in Anspruch 5 genannten allgemeinen Formel (1) durch Hydrolyse mit wäßriger Mineralsäure in das 3,4,5,6-Tetrafluorphthalsäureanhydrid bzw. durch Alkoholyse mit alkoholischer Mineralsäure in die entsprechenden 3,4,5,6-Tetrafluorphthalsäurediester überführt und diese durch weitere Hydrolyse bzw. Decarborylierung in bekannter Weise in die 2,3,4,5-Tetrafluorbenzoesäure umwandelt.

## Patentansprüche (Patentansprüche für folgende(n) Vertragsstaat(en): ES)

1. Verfahren zur Herstellung von Verbindungen der allgemeinen Formel (1) 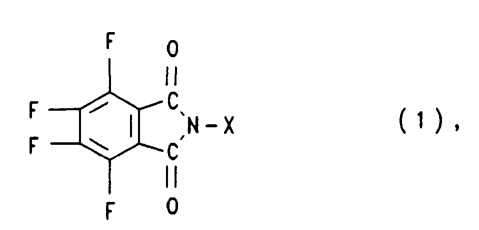 in welcher X den Rest 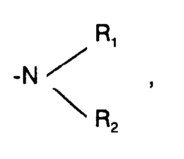 worin R₁, R₂ je ein Wasserstoffatom, eine Alkyl(C₁-C₁₀)-Gruppe, Arylgruppe, Alkyl(C₁-C₆)-CO-Gruppe oder Aryl-CO-Gruppe bedeuten, wobei die Aryl- bzw. Aryl-CO-Gruppen für R₁ und R₂ am aromatischen Kern durch Fluor- und/oder Chloratome und/oder Alkyl(C₁-C₄)-Gruppen substituiert sein können, oder R₁ und R₂ zusammen einen Phthaloylrest, der am aromatischen Ring durch 4 Chloratome oder 4 Fluoratome substituiert sein kann, vorzugsweise den Rest 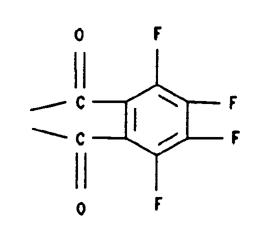 bedeuten, oder in welcher X den Rest 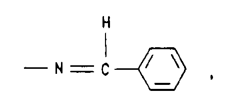 welcher am aromatischen Kern durch Fluor- und/oder Chloratome und/oder Alkyl(C₁-C₄)-Gruppen substituiert sein kann, bedeutet, dadurch gekennzeichnet, daß man 1 Mol 3,4,5,6-Tetrachlorphthalsäureanhydrid mit der mindestens äquimolaren Menge einer Stickstoffverbindung der allgemeinen Formel (2) 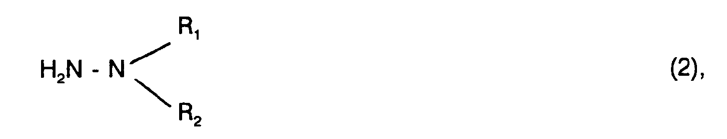 in welcher R₁ und R₂ die vorstehend genannten Bedeutungen haben, in wäßrig-alkoholischem Medium, in Eisessig, in etwa 90 bis 100 %iger Schwefelsäure oder in Oleum bei Temperaturen (in Abhängigkeit vom angewandten Medium) von etwa 100 bis etwa 220°C zum entsprechenden N′-substituierten N-Amino-3,4,5,6-tetrachlorphthalimid der allgemeinen Formel (3) 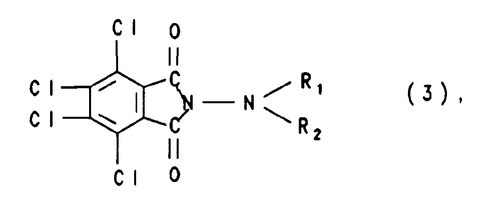 in welcher R₁ und R₂ die vorstehend genannten Bedeutungen haben, umsetzt, das so erhaltene Imid direkt oder nach erfolger Umsetzung mit der mindestens äquimolaren Menge Benzaldehyd, welcher am aromatischen Kern durch Fluor- und/oder Chloratome und/oder Alkyl(C₁-C₄)-Gruppen substituiert sein kann, zur entsprechenden Benzalverbindung, oder nach erfolgter Acylierung mit einem Alkyl(C₁-C₆)-CO-halogenid, Carbonsäureanhydrid der allgemeinen Formel Alkyl(C₁-C₆)-CO-O-OC-(C₁-C₆)Alkyl, Aryl-CO-halogenid oder Phthalsäureanhydrid, das am aromatischen Ring durch 4 Chloratome oder 4 Fluoratome substituiert sein kann, in einem polaren aprotischen Lösungsmittel mit Kalium-, Rubidium- oder Cäsiumfluorid oder Mischungen daraus bei Temperaturen von etwa 50 bis etwa 230°C in Gegenwart oder Abwesenheit eines Phasentransferkatalysators umsetzt.

2. Verfahren nach Anspruch 1, dadurch gekennzeichnet, daß man die Umsetzung mit Kalium-, Rubidium- oder Cäsiumfluorid oder Mischungen daraus bei Temperaturen von etwa 90 bis etwa 140°C vornimmt.

3. Verfahren nach mindestens einem der Ansprüche 1 und 2, dadurch gekennzeichnet, daß man die genannten Alkalimetallfluoride in Mengen von 100 bis etwa 500 Mol.-% pro auszutauschendem Chloratom einsetzt.

4. Verfahren nach mindestens einem der Ansprüche 1 bis 3, dadurch gekennzeichnet, daß man die Fluorierung in Dimethylformamid, Dimethylacetamid, Dimethylsulfoxid, Tetramethylensulfoxid, Dimethylsulfon, Diphenylsulfoxid, Sulfolan, N-Methylpyrrolidon oder 1,3-Dimethylimidazolidin-2-on als polar aprotischem Lösungsmittel vornimmt.

5. Verfahren nach mindestens einem der Ansprüche 1 bis 4, dadurch gekennzeichnet, daß man die Fluoridierung in Gegenwart eines quartären Ammonium- oder Phosphoniumsalzes als Phasentransferkatalysator vornimmt.

6. Verfahren nach mindestens einem der Ansprüche 1 bis 5, dadurch gekennzeichnet, daß man die Fluorierung in Gegenwart von Tetraalkyl-(C₁-C₁₈)-ammoniumchloriden oder -bromiden, Tetraalkyl(C₁-C₁₈)-phosphoniumchloriden oder -bromiden, Tetraphenylphosphoniumchlorid oder -bromid, [(Phenyl)ₘ(alkyl(C₁-C₁₈)ₙ]-phosphoniumchloriden oder -bromiden, wobei m = 1 bis 3, n = 3 bis 1 und m + n = 4 ist, als Phasentransferkatalysatoren vornimmt.

7. Verfahren nach mindestens einem der Ansprüche 1 bis 6, dadurch gekennzeichnet, daß man die Phasentransferkatalysatoren in Mengen von etwa 0,1 bis etwa 50 Mol.-%, bezogen auf das N′-substituierte N-Amino-3,4,5,6-tetrachlorophthalimid, einsetzt.

8. Verfahren nach mindestens einem der Ansprüche 1 bis 7, dadurch gekennzeichnet, daß man bei Atmosphärendruck, Über- oder Unterdruck arbeitet.

9. Verfahren zur Herstellung von 2,3,4,5-Tetrafluorbenzoesäure, dadurch gekennzeichnet, daß man die nach Ansprüchen 1 bis 8 hergestellten Verbindungen der in Anspruch 1 genannten allgemeinen Formel (1) durch Hydrolyse mit wäßriger Mineralsäure in das 3,4,5,6-Tetrafluorphthalsäureanhydrid bzw. durch Alkoholyse mit alkoholischer Mineralsäure in die entsprechenden 3,4,5,6-Tetrafluorphthalsäurediester überführt und diese durch weitere Hydrolyse bzw. Decarboxylierung in bekannter Weise in die 2,3,4,5-Tetrafluorbenzoesäure umwandelt.

## Claims (Claims for the following Contracting State(s): BE, CH, DE, FR, GB, IT, LI, NL)

1. A compound of the formula 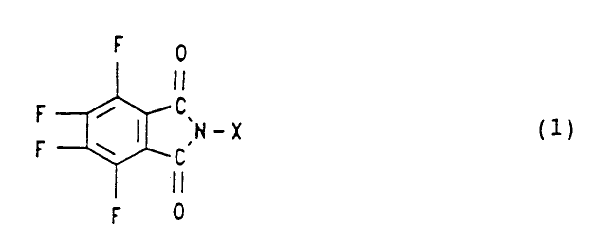 in which X is the radical 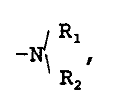 where R₁ and R₂ are in each case a hydrogen atom, an alkyl(C₁-C₁₀) group, aryl group, alkyl(C₁-C₆)-CO group or aryl-CO group, where the aryl, or aryl-CO, groups in the case of R₁ and R₂ can be substituted on the aromatic ring by fluorine and/or chlorine atoms and/or alkyl(C₁-C₄) groups, or R₁ and R₂ together are a phthaloyl radical which can be substituted on the aromatic ring by 4 chlorine atoms or 4 fluorine atoms, preferably the radical 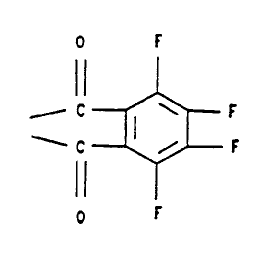 or in which X is the radical 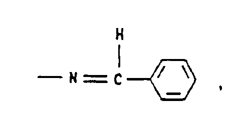 which can be substituted on the aromatic ring by fluorine and/or chlorine atoms and/or alkyl(C₁-C₄) groups.

2. A compound of the formula

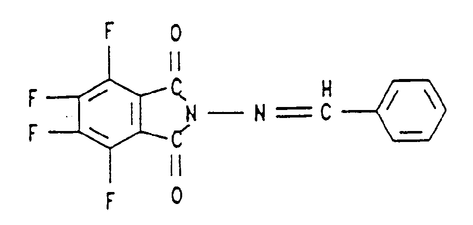

3. A compound of the formula

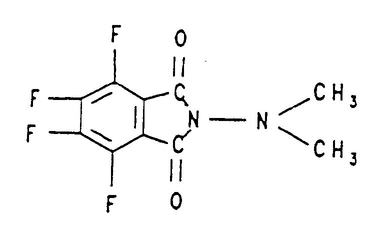

4. A compound of the formula

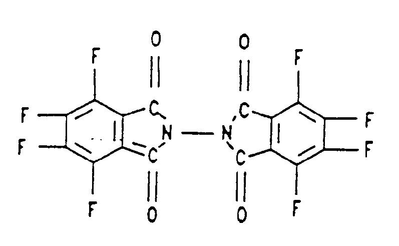

5. A process for the preparation of a compound of the formula (1) 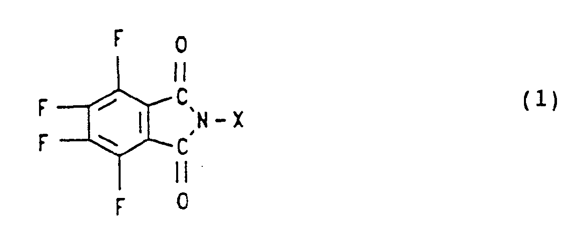 in which X is the radical 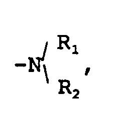 where R₁ and R₂ are in each case a hydrogen atom, an alkyl(C₁-C₁₀) group, aryl group, alkyl(C₁-C₆)-CO group or aryl-CO group, where the aryl, or aryl-CO, groups in the case of R₁ and R₂ can be substituted on the aromatic ring by fluorine and/or chlorine atoms and/or alkyl(C₁-C₄) groups, or R₁ and R₂ together are a phthaloyl radical which can be substituted on the aromatic ring by 4 chlorine atoms or 4 fluorine atoms, preferably the radical 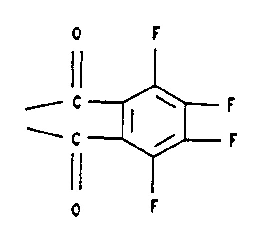 or in which X is the radical 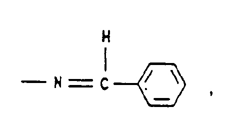 which can be substituted on the aromatic ring by fluorine and/or chlorine atoms and/or alkyl(C₁-C₄) groups, which comprises reacting 1 mol of 3,4,5,6-tetrachlorophthalic anhydride with an at least equimolar amount of a nitrogen compound of the formula (2) 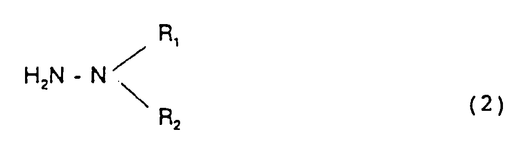 in which R₁ and R₂ have the abovementioned meanings, in an aqueous-alcoholic medium, in glacial acetic acid, in approximately 90 to 100% strength sulfuric acid or in oleum at temperatures (depending on the medium used) of approximately 100 to approximately 220°C, to give the corresponding N′-substituted N-amino-3,4,5,6-tetrachlorophthalimide of the formula (3) 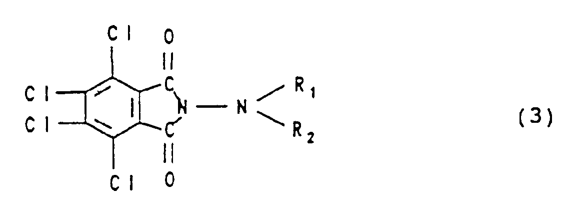 in which R₁ and R₂ have the abovementioned meanings, and reacting the resulting imide with potassium fluoride, rubidium fluoride or cesium fluoride or mixtures of these at temperatures of approximately 50 to approximately 230°C in the presence or absence of a phase-transfer catalyst, in a polar aprotic solvent, directly or after prior reaction with an at least equimolar amount of benzaldehyde which can be substituted on the aromatic ring by fluorine and/or chlorine atoms and/or alkyl(C₁-C₄) groups to give the corresponding benzal compound, or after prior acylation with an alkyl(C₁-C₆)-CO halide, carboxylic anhydride of the formula alkyl(C₁-C₆)-CO-O-OC-(C₁-C₆)alkyl, aryl-CO halide or phthalic anhydride which can be substituted on the aromatic ring by 4 chlorine atoms or 4 fluorine atoms.

6. The process as claimed in claim 5, wherein the reaction with potassium fluoride, rubidium fluoride or cesium fluoride, or mixtures of these, is carried out at temperatures of approximately 90 to approximately 140°C.

7. The process as claimed in at least one of claims 5 and 6, wherein the abovementioned alkali metal fluorides are employed in amounts of 100 to approximately 500 mol% per chlorine atom to be exchanged.

8. The process as claimed in at least one of claims 5 to 7, wherein the fluorination is carried out in dimethylformamide, dimethylacetamide, dimethyl sulfoxide, tetramethylene sulfoxide, dimethyl sulfone, diphenyl sulfoxide, diphenyl sulfone, sulfolane, N-methylpyrrolidone or 1,3-dimethylimidazolidin-2-one as polar aprotic solvent.

9. The process as claimed in at least one of claims 5 to 8, wherein the fluorination is carried out in the presence of a quaternary ammonium or phosphonium salt as phase-transfer catalyst.

10. The process as claimed in at least one of claims 5 to 9, wherein the fluorination is carried out in the presence of tetraalkyl(C₁-C₁₈)ammonium chlorides or tetraalkyl(C₁-C₁₈)ammonium bromides, tetraalkyl-(C₁-C₁₈)phosphonium chlorides or tetraalkyl(C₁-C₁₈)-phosphonium bromides, tetraphenylphosphonium chloride or tetraphenylphosphonium bromide, [(phenyl)ₘ(alkyl(C₁-C₁₈))ₙ]-phosphonium chlorides or [(phenyl)ₘ(alkyl(C₁-C₁₈))ₙ]-phosphonium bromides, where m is 1 to 3, n is 3 to 1 and m + n is 4, as phase-transfer catalysts.

11. The process as claimed in at least one of claims 5 to 10, wherein the phase-transfer catalysts are employed in amounts of approximately 0.1 to approximately 50 mol%, based on the N′-substituted N-amino-3,4,5,6-tetrachlorophthalimide.

12. The process as claimed in at least one of claims 5 to 11, wherein the pressure at which it is carried out is atmospheric pressure, superatmospheric pressure or subatmospheric pressure.

13. A process for the preparation of 2,3,4,5-tetrafluorobenzoic acid, which comprises converting the compounds of the formula (1) mentioned in claim 5, which have been prepared in accordance with claims 5 to 12, into 3,4,5,6-tetra-fluorophthalic anhydride by hydrolysis with aqueous mineral acid, or into the corresponding 3,4,5,6-tetrafluorophthalic acid diesters by alcoholysis with alcoholic mineral acid, and converting these products in a known manner into 2,3,4,5-tetrafluorobenzoic acid by further hydrolysis or by decarboxylation.

## Claims (Claims for the following Contracting State(s): ES)

1. A process for the preparation of a compound of the formula (1) 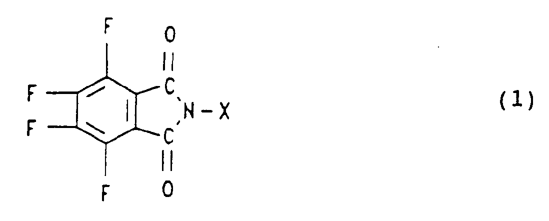 in which X is the radical 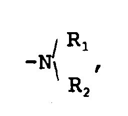 where R₁ and R₂ are in each case a hydrogen atom, an alkyl(C₁-C₁₀) group, aryl group, alkyl(C₁-C₆)-CO group or aryl-CO group, where the aryl, or aryl-CO, groups in the case of R₁ and R₂ can be substituted on the aromatic ring by fluorine and/or chlorine atoms and/or alkyl(C₁-C₄) groups, or R₁ and R₂ together are a phthaloyl radical which can be substituted on the aromatic ring by 4 chlorine atoms or 4 fluorine atoms, preferably the radical 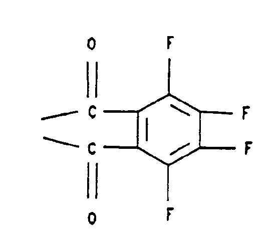 or in which X is the radical 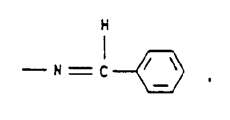 which can be substituted on the aromatic ring by fluorine and/or chlorine atoms and/or alkyl(C₁-C₄) groups, which comprises reacting 1 mol of 3,4,5,6-tetrachlorophthalic anhydride with an at least equimolar amount of a nitrogen compound of the formula (2) 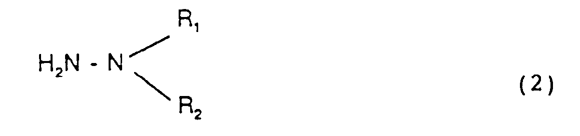 in which R₁ and R₂ have the abovementioned meanings, in an aqueous-alcoholic medium, in glacial acetic acid, in approximately 90 to 100% strength sulfuric acid or in oleum at temperatures (depending on the medium used) of approximately 100 to approximately 220°C, to give the corresponding N′-substituted N-amino-3,4,5,6-tetrachlorophthalimide of the formula (3) 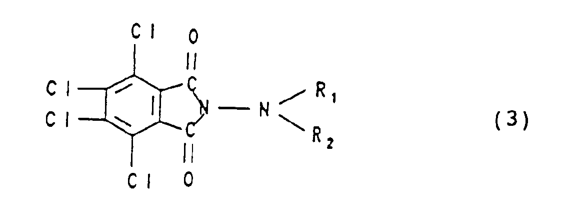 in which R₁ and R₂ have the abovementioned meanings, and reacting the resulting imide with potassium fluoride, rubidium fluoride or cesium fluoride or mixtures of these at temperatures of approximately 50 to approximately 230°C in the presence or absence of a phase-transfer catalyst, in a polar aprotic solvent, directly or after prior reaction with an at least equimolar amount of benzaldehyde which can be substituted on the aromatic ring by fluorine and/or chlorine atoms and/or alkyl(C₁-C₄) groups to give the corresponding benzal compound, or after prior acylation with an alkyl(C₁-C₆)-CO halide, carboxylic anhydride of the formula alkyl(C₁-C₆)-CO-O-OC-(C₁-C₆)alkyl, aryl-CO halide or phthalic anhydride which can be substituted on the aromatic ring by 4 chlorine atoms or 4 fluorine atoms.

2. The process as claimed in claim 1, wherein the reaction with potassium fluoride, rubidium fluoride or cesium fluoride, or mixtures of these, is carried out at temperatures of approximately 90 to approximately 140°C.

3. The process as claimed in at least one of claims 1 and 2, wherein the abovementioned alkali metal fluorides are employed in amounts of 100 to approximately 500 mol% per chlorine atom to be exchanged.

4. The process as claimed in at least one of claims 1 to 3, wherein the fluorination is carried out in dimethylformamide, dimethylacetamide, dimethyl sulfoxide, tetramethylene sulfoxide, dimethyl sulfone, diphenyl sulfoxide, diphenyl sulfone, sulfolane, N-methylpyrrolidone or 1,3-dimethylimidazolidin-2-one as polar aprotic solvent.

5. The process as claimed in at least one of claims 1 to 4, wherein the fluorination is carried out in the presence of a quaternary ammonium or phosphonium salt as phase-transfer catalyst.

6. The process as claimed in at least one of claims 1 to 5, wherein the fluorination is carried out in the presence of tetraalkyl(C₁-C₁₈)-ammonium chlorides or tetraalkyl(C₁-C₁₈)ammonium bromides, tetraalkyl-(C₁-C₁₈)phosphonium chlorides or tetraalkyl(C₁-C₁₈)-phosphonium bromides, tetraphenylphosphonium chloride or tetraphenylphosphonium bromide, [(phenyl)ₘ(alkyl(C₁-C₁₈))ₙ]-phosphonium chlorides or [(phenyl)ₘ(alkyl(C₁-C₁₈))ₙ]-phosphonium bromides, where m is 1 to 3, n is 3 to 1 and m + n is 4, as phase-transfer catalysts.

7. The process as claimed in at least one of claims 1 to 6, wherein the phase-transfer catalysts are employed in amounts of approximately 0.1 to approximately 50 mol%, based on the N′-substituted N-amino-3,4,5,6-tetrachlorophthalimide.

8. The process as claimed in at least one of claims 1 to 7, wherein the pressure at which it is carried out is atmospheric pressure, superatmospheric pressure or subatmospheric pressure.

9. A process for the preparation of 2,3,4,5-tetrafluorobenzoic acid, which comprises converting the compounds of the formula (1) mentioned in claim 1, which have been prepared in accordance with claims 1 to 8, into 3,4,5,6-tetra-fluorophthalic anhydride by hydrolysis with aqueous mineral acid, or into the corresponding 3,4,5,6-tetrafluorophthalic acid diesters by alcoholysis with alcoholic mineral acid, and converting these products in a known manner into 2,3,4,5-tetrafluorobenzoic acid by further hydrolysis or by decarboxylation.

## Revendications (Revendications pour l'(les) Etat(s) contractant(s) suivant(s): BE, CH, DE, FR, GB, IT, LI, NL)

1. Composés de formule générale 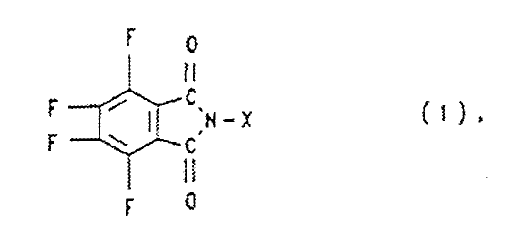 dans laquelle X représente le reste 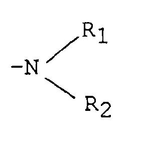 dans lequel R₁ et R₂ représentent chacun un atome d'hydrogène, un groupe alkyle en C₁ à C₁₀, un groupe aryle, un groupe alkyle (en C₁ à C₆)-CO-, ou un groupe aryl-CO-, les groupes aryl ou aryl-CO représentés par R₁ et R₂ pouvant être substitués sur le noyau par des atomes de fluor et/ou par des atomes de chlore et/ou par des groupes alkyles en C₁ à C₄, ou bien R₁ et R₂ forment ensemble un reste phtaloyle, qui peut être substitué sur le noyau aromatique par 4 atomes de chlore ou par 4 atomes de fluor, avantageusement X représente le reste 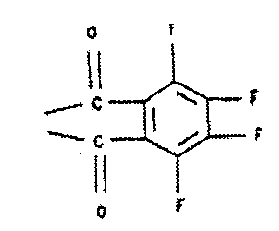 ou bien X représente le reste 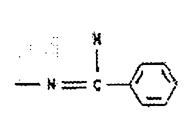 qui peut être substitué sur le noyau aromatique; par des atomes de fluor et/ou par des atomes de chlore et/ou par des groupes alkyles en C₁ à C₄.

2. Composé de formule

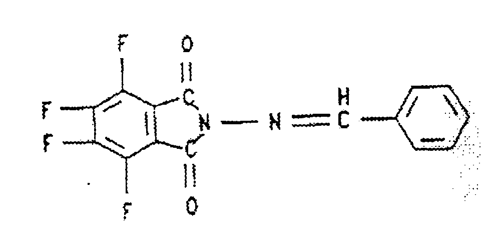

3. Composé de formule

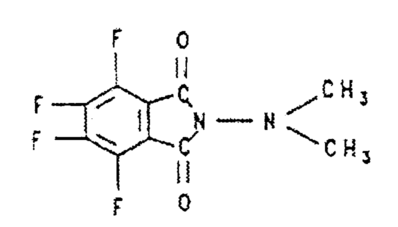

4. Composé de formule

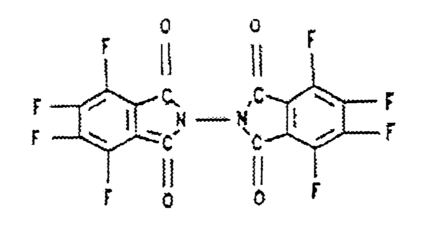

5. Procédé de préparation de composés de formule générale (1) : 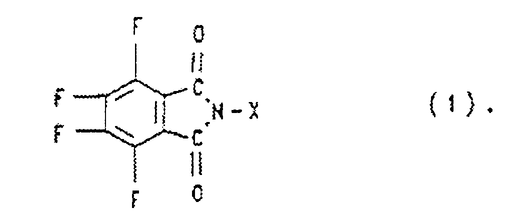 dans laquelle X représente le reste 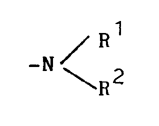 dans lequel R₁ et R₂ représentent chacun un atome d'hydrogène, un groupe alkyle en C₁ à C₁₀ un groupe aryle, un groupe alkyl (en C₁ à C₆)-CO- ou un groupe aryle - CO, les groupes aryles ou aryl-CO représentés par R₁, R₂ pouvant être substitués sur le noyau aromatique par des atomes de fluor et/ou par des atomes de chlore et/ou par des groupes alkyles (en C₁ à C₄), ou bien R₁, R₂ forment ensemble un reste phtaloyle, qui peut être substitué sur le noyau aromatique par 4 atomes de chlore ou par 4 atomes de fluor, avantageusement le reste : 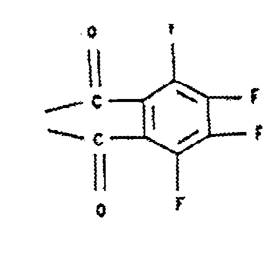 ou bien X représente le reste 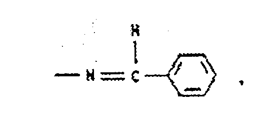 (qui peut être substitué sur le noyau aromatique par des atomes de fluor et/ou par des atomes de chlore et/ou par des groupes alkyles (en C₁ à C₄)), procédé caractérisé en ce qu'on fait réagir une mole d'anhydride de l'acide 3,4,5,6-tétrachlorophtalique avec au moins la quantité équimolaire d'un composé azoté de formule générale (2) 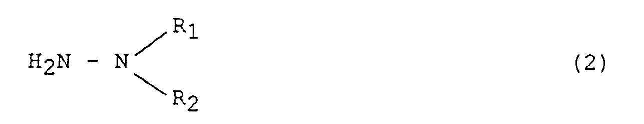 (dans laquelle R₁ et R₂ ont le sens précité) en un milieu hydro-alcoolique, dans de l'acide acétique cristallisable, dans de l'acide sulfurique à environ 90 à 100 % ou dans de l'oléum, à des températures (qui dépendent du milieu appliqué) d'environ 100 à environ 220°C pour obtenir le N-amino-3,4,5,6-tétrachlorophtalimide, substitué en N′, correspondant, de formule générale (3) 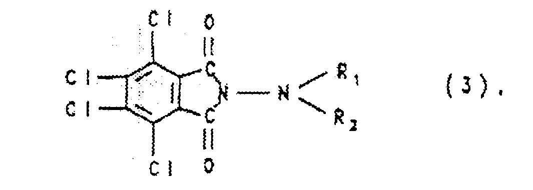 (dans laquelle R₁ et R₂ ont les sens précités), et l'on soumet l'imide ainsi obtenu, directement ou après une réaction avec une quantité au moins équimolaire de benzaldéhyde (qui peut être substitué sur le noyau aromatique par des atomes de fluor et/ou par des atomes de chlore et/ou par des groupes alkyles en C₁ à C₄)) pour obtenir le composé de type benzal correspondant, ou après une acylation effectuée avec un halogénure de CO-alkyle (en C₁ à C₆), avec un anhydride d'acide carboxylique de formule générale
alkyl (en C₁-C₆)-CO-O-OC-alkyle (en C₁-C₆)
avec un halogénure de CO-aryle ou avec l'anhydride de l'acide phtalique (qui peut être substitué sur des noyaux aromatiques par 4 atomes de chlore ou par 4 atomes de fluor) à une réaction, effectuée dans un solvant aprotique polaire avec du fluorure de potassium, du fluorure de rubidium, du fluorure de césium ou leurs mélanges, à des températures d'environ 50 à environ 230°C, en présence ou en l'absence d'un catalyseur de transfert de phase.

6. Procédé selon la revendication 5 caractérisé en ce qu'on effectue la réaction avec du fluorure de potassium, du fluorure de rubidium, ou du fluorure de césium ou leurs mélanges à des températures d'environ 90 à environ 140°C.

7. Procédé selon l'une au moins des revendications 5 et 6, caractérisé en ce qu'on utilise les fluorures de métaux alcalins cités en des quantités de 100 à environ 500 moles % par atome de chlore à remplacer.

8. Procédé selon l'une au moins des revendications 5 à 7, caractérisé en ce qu'on effectue la fluoration dans du diméthylformamide, dans du diméthyl acétamide, dans du diméthylsulfoxyde, dans du tétraméthylène sulfoxyde, dans de la diméthylsulfone, dans du diphénylsulfoxyde, dans de la diphénylsulfone, dans du sulfolane, dans de la N-méthylpyrrolidone ou dans de la 1,3-diméthylimidazolidine-2-one comme solvant aprotique polaire.

9. Procédé selon l'une au moins des revendications 5 à 8, caractérisé en ce qu'on effectue la fluoration en présence d'un sel d'ammonium quaternaire ou de phosphonium quaternaire comme catalyseur de transfert de phase.

10. Procédé selon l'une au moins des revendications 5 à 9, caractérisé en ce qu'on effectue la fluoration en présence de chlorures ou de bromures de tétralkyl (en C₁ à C₁₈)-ammonium, de chlorures ou de bromures de tétraalkyl (en C₁ à C₁₈)-phosphonium, de chlorure ou de bromure de tétraphénylphosphonium, de chlorures ou de bromures de [(phényl)ₘ(alkyl(en C₁ à C₁₈))ₙ]-phosphonium, dans lesquels m vaut 1 à 3, n vaut 3 à 1 et la somme (m+n) vaut 4, comme catalyseur de transfert de phase.

11. Procédé selon l'une au moins des revendications 5 à 10, caractérisé en ce qu'on utilise les catalyseurs de transfert de phase en des quantités d'environ 0,1 à environ 50 moles %, par rapport au N-amino-3,4,5,6-tétrachlorophtalimide substitué en N′.

12. Procédé selon l'une au moins des revendications 5 à 11, caractérisé en ce qu'on travaille sous la pression atmosphérique, ou sous une pression supérieure ou inférieure à la pression atmosphérique.

13. Procédé de préparation de l'acide 2,3,4,5-tétrafluorobenzoïque, caractérisé en ce qu'on soumet les composés préparés selon les revendications 5 à 12 et répondant à la formule générale (1) citée à la revendication 5 à une transformation par hydrolyse avec de l'acide minéral aqueux en l'anhydride d'acide 3,4,5,6-tétrafluorophtalique ou à une transformation par alcoolyse avec de l'acide minéral alcoolique en ledit ester correspondant de l'acide 3,4,5,6-tétrafluorophtalique et l'on transforme ce composé, par une continuation de l'hydrolyse ou par une décarboxylation, de façon connue, en l'acide 2,3,4,5-tétrafluorobenzoïque.

## Revendications (Revendications pour l'(les) Etat(s) contractant(s) suivant(s): ES)

1. Procédé pour préparer des composés de formule générale (1) : 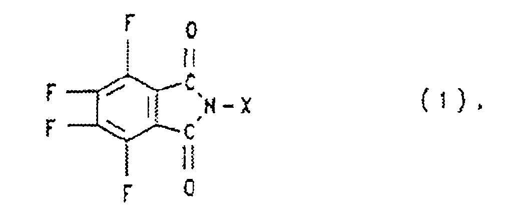 dans laquelle X représente le reste 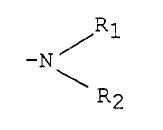 dans lequel R₁ et R₂ représentent chacun un atome d'hydrogène, un groupe alkyle en C₁ à C₁₀, un groupe aryle, un groupe alkyl (en C₁ à C₆ )-CO-, ou un groupe aryl-CO-, les groupes aryl ou aryl-CO représentés par R₁ et R₂ pouvant être susbtitués sur le noyau aromatique par des atomes de fluor et/ou par des atomes de chlore et/ou par des groupes alkyles en C₁ à C₄, ou bien R₁ et R₂ forment ensemble un reste phtaloyle, qui peut être substitué sur le noyau aromatique par 4 atomes de chlore ou par 4 atomes de fluor, avantageusement le reste 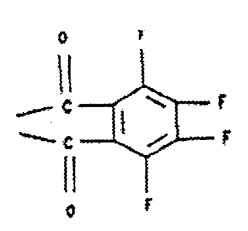 ou bien X représente le reste 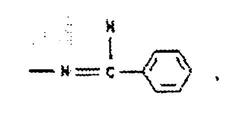 (qui peut être substitué sur le noyau aromatique par des atomes de fluor et/ou par des atomes de chlore et/ou par des groupes alkyles (en C₁ à C₄)), procédé caractérisé en ce qu'on fait réagir une mole d'anhydride de l'acide 3,4,5,6-tétrachlorophtalique avec au moins la quantité équimolaire d'un composé azoté de formule générale (2) 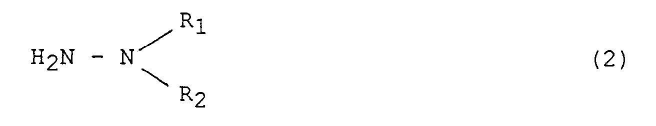 (dans laquelle R₁ et R₂ ont le sens précité) dans un milieu hydro-alcoolique, dans de l'acide acétique cristallisable, dans de l'acide sulfurique à environ 90 à 100 % ou dans de l'oléum, à des températures (qui dépendent du milieu appliqué) d'environ 100 à environ 220°C pour obtenir le N-amino-3,4,5,6-tétrachlorophtalimide, substitué en N′, correspondant, et répondant à la formule générale (3) 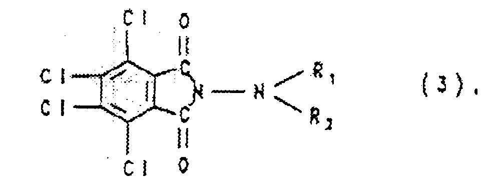 (dans laquelle R₁ et R₂ ont les sens précités), et l'on soumet l'imide ainsi obtenu, directement ou après une réaction avec une quantité au moins équimolaire de benzaldéhyde (qui peut être substitué sur le noyau aromatique par des atomes de fluor et/ou par des atomes de chlore et/ou par des groupes alkyles en C₁ à C₄)) pour obtenir le composé de type benzal correspondant, ou après une acylation réalisée avec un halogénure de CO-alkyle (en C₁ à C₆), (ou halogénure d'alkyl carbonyle), avec un anhydride d'acide carboxylique de formule générale
alkyl(C₁-C₆)-CO-O-OC-alkyle (en C₁-C₆)
avec un halogénure de CO-aryle (ou halogénure d'aryl-carbonyle) ou avec l'anhydride de l'acide phtalique, qui peut être substitué sur le noyau aromatique par 4 atomes de chlore ou par 4 atomes de fluor, à une réaction, effectuée dans un solvant aprotique polaire avec du fluorure de potassium, du fluorure de rubidium, du fluorure de césium ou avec le mélange de ces fluorures, à des températures d'environ 50 à environ 230°C, en présence ou en l'absence d'un catalyseur de transfert de phase.

2. Procédé selon la revendication 1, caractérisé en ce qu'on effectue la réaction avec le fluorure de potassium, de rubidium ou de césium ou avec des mélanges de ces fluorures en opérant à des températures d'environ 90 à environ 140°C.

3. Procédé selon l'une au moins des revendications 1 et 2, caractérisé en ce qu'on utilise les fluorures de métaux alcalins cités en des quantités de 100 à environ 500 moles % pour un atome de chlore à remplacer.

4. Procédé selon l'une au moins des revendications 1 à 3, caractérisé en ce qu'on effectue la fluoration dans du diméthylformamide, dans du diméthyl acétamide, dans du diméthylsulfoxyde, dans du tétraméthylène sulfoxyde, dans de la diméthylsulfone, dans du diphénylsulfoxyde, dans du sulfolane, dans de la N-méthylpyrrolidone ou dans de la 1,3-diméthylimidazolidine-2-one comme solvant aprotique polaire.

5. Procédé selon l'une au moins des revendications 1 à 4, caractérisé en ce qu'on effectue la fluoration en présence d'un sel d'ammonium quaternaire ou de phosphonium quaternaire comme catalyseur de transfert de phase.

6. Procédé selon l'une au moins des revendications 1 à 5, caractérisé en ce qu'on effectue la fluoration en présence de chlorures ou de bromures de tétra-alkyl (en C₁ à C₁₈)-ammoniums, de chlorures ou de bromures de tétra-alkyl (en C₁ à C₁₈)-phosphoniums, de chlorure ou de bromure de tétraphénylphosphonium, de chlorures ou de bromures de [(phényl)ₘ(alkyl(en C₁ à C₁₈))ₙ]-phosphonium, formule dans laquelle m vaut 1 à 3, n vaut 3 à 1 et la somme (m+n) vaut 4, comme catalyseur de transfert de phase.

7. Procédé selon l'une au moins des revendications 1 à 6, caractérisé en ce qu'on utilise les catalyseurs de transfert de phase en des quantités d'environ 0,1 à environ 50 moles %, par rapport au N-amino-3,4,5,6-tétrachlorophtalimide substitué en N′.

8. Procédé selon l'une au moins des revendications 1 à 7, caractérisé en ce qu'on travaille sous la pression atmosphérique, ou sous une pression supérieure ou inférieure à la pression atmosphérique.

9. Procédé pour produire de l'acide 2,3,4,5-tétrafluorobenzoïque, caractérisé en ce qu'on soumet les composés préparés selon les revendications 1 à 8 et répondant à la formule générale (1) citée à la revendication 1, à une transformation par hydrolyse avec de l'acide minéral aqueux, ce qui donne l'anhydride d'acide 3,4,5,6-tétrafluorophtalique ou bien par alcoolyse avec de l'acide minéral alcoolique, ce qui donne l'ester correspondant de l'acide 3,4,5,6-tétrahydrophtalique et l'on transforme ce composé, par une poursuite de l'hydrolyse ou par une décarboxylation, de façon connue, en l'acide 2,3,4,5-tétrafluorobenzoïque.
